# FASCICULE DE BREVET EUROPEEN

(11) **EP 0 705 099 B1**
(45) Date de publication et mention de la délivrance du brevet: **24.10.2001**
(21) Numéro de dépôt: 94919708.1
(22) Date de dépôt: 14.06.1994
(51) Int. Cl.: A61K 31/165, A61P 11/00

(54) **UTILISATION DU MODAFINIL POUR LE TRAITEMENT DES APNEES DU SOMMEIL ET TROUBLES VENTILATOIRES D'ORIGINE CENTRALE**
Verwendung vonModafinil zur Behandlung von Atemstillstand im Schlaf und zentral erregten Atembeschwerden
USE OF MODAFINIL FOR THE TREATMENT OF SLEEP APNOEA AND VENTILATION PROBLEMS OF CENTRAL ORIGIN

(30) Priorité: 22.06.1993 FR 9307555
(43) Date de publication de la demande: 10.04.1996
(73) Titulaire: LABORATOIRE L. LAFON, 94701 Maisons Alfort (FR)
(72) Inventeur: LAURENT, Philippe, F-69600 Oullins (FR)
(74) Mandataire: Le Guen, Gérard
(86) Numéro de dépôt international: FR9400711
(87) Numéro de publication internationale: WO9500132

(56) Documents cités:
- EP-A- 0 462 004
- FR-A- 2 385 693
- FR-A- 2 684 875
- NEUROPHYSIOL. CLIN., vol.23, no.1, 1993 pages 23 - 33 C. GUILLEMINAULT ET AL. 'Daytime somnolescence: therapeutic approaches'

## Description

La présente invention concerne une nouvelle utilisation en thérapeutique du modafinil.

Le modafinil ou benzhydrylsulfinyl acétamide est un composé de formule :

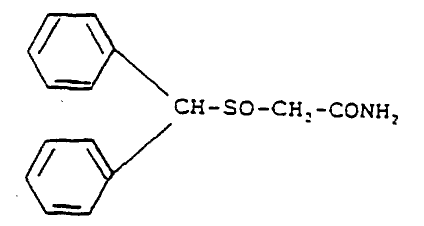

Ce composé et son application thérapeutique comme agent actif sur le système nerveux central ont été décrits dans la demande de brevet FR-A-2 385 693. Cette demande décrit des propriétés pharmacologiques montrant des effets pharmacologiques éveillants et antidépresseurs. Des résultats très favorables sont mentionnés pour le traitement des asthénies des personnes âgées. De plus le produit est décrit comme utile dans le traitement des dyskinésies tardives des neuroleptiques. La demande FR-A-2 663 225 décrit des propriétés thérapeutiques utiles pour le traitement des maladies neurodégénératives des voies dopaminergiques du système nerveux central tel que la maladie de Parkinson. D'autre part, la demande de brevet français FR-A- 2 684 875 décrit des propriétés utiles pour le traitement des séquelles des accidents ischémiques cérébraux.

On a maintenant découvert que le modafinil possède un effet bénéfique vis-à-vis des apnées survenant au cours des syndromes d'apnée du sommeil.

La présente invention a en conséquence pour objet l'utilisation du modafinil pour la fabrication d'un médicament ayant un effet bénéfique sur la survenue des apnées caractérisant les syndromes d'apnée du sommeil, et d'une manière plus générale les troubles ventilatoires d'origine centrale.

Le médicament anti-apnée contenant le modafinil peut être présenté notamment sous une forme convenant pour l'administration par voie orale. Généralement la dose administrée peut être de 1 mg/kg à 100 mg/kg.

Il est rappelé qu'une apnée est définie comme une interruption du flux aérien naso-buccal excédant une durée fixée arbitrairement à 10 secondes (*Guilleminault C., Tilkian A*., *Dément WC. : Ann. Rev. Med.,* 1976, 27 : 465-484). Trois types d'apnées sont à distinguer : les apnées d'origine centrale, les apnées par obstruction des voies respiratoires, et les apnées dites mixtes associant les deux causes centrales et obstructives. Les syndromes d'apnée du sommeil sont classiquement définis par un index d'apnée supérieur à 5 apnées par heure de sommeil. De façon plus récente, les syndromes sont définis par un index d'apnée et d'hypopnée supérieur à 10 évènements par heure de sommeil.

On donnera ci-après le résultat d'un essai clinique mettant en évidence les effets anti-apnées du modafinil chez l'homme.

6 sujets, des deux sexes, suivis en service de consultation spécialisée et souffrant de syndrome d'apnée du sommeil d'origine centrale et mixte ont été inclus dans un essai clinique pilote en simple aveugle et comparant l'effet du modafinil aux traitements habituels tels que la CPAP (nasal continuous positive airway pressure ). Les patients ont été diagnostiqués par méthode d'enregistrement polysomnographique comportant l'identification des stades de sommeil et la mesure de la durée de chaque stade, la détection des apnées, et la mesure des conséquences de ces apnées sur les paramètres gazométriques artériels, la fréquence cardiaque et la pression artérielle. L'identification des stades de sommeils a été effectuée à l'aide d'enregistrements électro-encéphalographiques (EEG), électromyographiques (EMG), électro-oculographique (EOG). L'activité ventilatoire a été déterminée à l'aide de thermistors. La saturation oxyhémoglobinée a été mesurée par mesure oxymétrique transcutanée. Enfin les autres paramètres gazométriques ont été mesurés à l'aide d'un analyseur automatique de gaz de type Corning. Les signes cliniques tels que la somnolence diurne et la qualité du sommeil nocturne ont été évalués à l'aide d'échelle d'évaluation clinique et d'agenda de sommeil remplis par le patient et l'investigateur clinique. Le modafinil a été administré pendant le jour, de préférence le matin et en début d'après-midi.

De façon inattendue, les résultats rapportent un effet sur le nombre d'apnées nocturnes survenant au cours du sommeil et d'hypopnée. Ce nombre diminue de façon significative chez 4 sujets sur 6 après 1 mois de traitement, et chez un sujet sur 6 les apnées disparaissent complètement des enregistrements après traitement. L'amélioration portant sur le nombre d'apnées survenant au cours du sommeil semble persister plusieurs jours après l'interruption du traitement. Les doses de modafinil administrées par jour étaient de 200 mg à 600 mg.

A titre d'exemple les résultats obtenus chez un patient sont présentés sur le tableau I comparant les résultats concernant les apnées avant et au cours de traitement par le modafinil.

Le traitement par modafinil a apporté une amélioration de la somnolence diurne et de la qualité du sommeil nocturne.

Les résultats cliniques observés chez l'homme permettent d'envisager l'application du médicament au traitement des apnées du sommeil et plus généralement aux troubles ventilatoires d'origine centrale.

**TABLEAU I**

| RESULTATS CONCERNANT LES APNEES | | |
|---|---|---|
| | Avant traitement | Pendant le traitement |
| NOMBRE TOTAL D'APNEES | 368 | 3 |
| Nombre d'apnées centrales | 269 | 2 |
| Nombre d'apnées périphé-riques | 59 | 1 |
| Nombre d'apnées mixtes | 40 | 0 |
| DUREE TOTALE DES APNEES | 7432 sec | 35 sec |
| Durée totale des apnées centrales | 5546 sec | 23 sec |
| Durée totale des apnées périphériques | 988 sec | 12 sec |
| Durée totale des apnées mixtes | 898 sec | O sec |

| REPARTITION GLOBALE DES APNEES | | |
|---|---|---|
| APNEES INFERIEURES A 10 SECONDES | | |
| Nombre | 2 | 0 |
| Durée totale | 18 sec | 0 sec |
| Durée moyenne | 9 sec | 0 sec |
| Durée totale/durée du sommeil | 0,06 % | 0 sec 0 % |
| APNEES EGALES OU SUPERIEU-RES A 10 SECONDES | | |
| Nombre | 366 | 3 |
| Durée totale | 7414 sec | 35 sec |
| Durée moyenne | 20 sec | 12 sec |
| Durée totale/durée du | | |
| sommeil | 26,23 % | 0,15 % |
| INDEX D'APNEES | 46,62 | 0,45 |
| TAUX D'APNEES | 26,3 % | 0,15 % |

## Revendications

1. Utilisation du modafinil pour la fabrication d'un médicament destiné au traitement des troubles ventilatoires d'origine centrale.

2. Utilisation du modafinil pour la fabrication d'un médicament ayant un effet anti-apnée du sommeil au cours de syndromes d'apnée du sommeil.

3. Utilisation selon la revendication 1 ou la revendication 2 pour la fabrication d'un médicament sous forme convenant pour l'administration par voie orale.

## Claims

1. Use of modafinil for the manufacture of a medicament intended for the treatment of ventilation disorders of central-nervous origin.

2. Use of modafinil for the manufacture of a medicament having an effect to control sleep apnoea in the course of sleep apnoea syndromes.

3. Use according to Claim 1 or 2 for the manufacture of a medicament in a form suitable for oral administration.

## Patentansprüche

1. Verwendung von Modafinil zur Herstellung eines Medikaments, das zur Behandlung von ventilatorischen Störungen zentraler Ursache bestimmt ist.

2. Verwendung von Modafinil zur Herstellung eines gegen die Schlafapnoe im Verlauf von Schlafapnoe-Syndromen gerichteten Medikaments.

3. Verwendung nach Anspruch 1 oder 2 zur Herstellung eines oral verabreichbaren Medikaments.
